# EUROPÄISCHE PATENTANMELDUNG

(11) **EP 2 848 227 A1**
(43) Veröffentlichungstag der Anmeldung: **18.03.2015**
(21) Anmeldenummer: 12876284.6
(22) Anmeldetag: 06.09.2012
(51) Int. Cl.: A61B 18/20, A61N 7/00, A61H 23/00

(54) **VERFAHREN ZUM ERNEUERN VON BIOLOGISCHEN GEWEBEN SOWIE VORRICHTUNG ZUR DURCHFÜHRUNG DIESES VERFAHRENS (VARIANTEN)**

(30) Priorität: 11.05.2012 EA 201200845
(71) Anmelder: Khomchanka, Uladzimir Valiantinavich, Minsk 220006 (BY); Gorbach, Dmitry Vladislavovich, Minsk 220102 (BY); Sukhadolau, Aliaksandr Valerjavich, Minsk 220117 (BY)
(72) Erfinder: Khomchanka, Uladzimir Valiantinavich, Minsk 220006 (BY); Gorbach, Dmitry Vladislavovich, Minsk 220102 (BY); Sukhadolau, Aliaksandr Valerjavich, Minsk 220117 (BY)
(74) Vertreter: Jeck, Anton
(86) Internationale Anmeldenummer: PCT/BY2012/000002
(87) Internationale Veröffentlichungsnummer: WO 2013/166577

(57) **Zusammenfassung**

Die Erfindung bezieht sich auf die Medizin und kann in der Chirurgie, darunter auch In der Schönheitschirurgie angewendet werden, z. B. zur Behandlung von trophischen und lange nicht heilenden Geschwüren, Dekubitalgeschwüren, Brandverletzungen, Narben usw. Der weitere Anwendungsbereich ist eine Verjüngung der biologischen Gewebe, darunter der Haut, mit unterschiedlicher Lokalisation. Es wird ein Verfahren zur biologischen Gewebeerneuerung mit Wiederherstellung der Leistungsmerkmale, der Eigenschaften und der Strukturen der Gewebe vorgeschlagen. Nach diesem Verfahren werden die Gewebe einer mechanischen Verletzung mit einem vorgegebenen Schweregrad ausgesetzt, Indem wenigstens ein Interferenzgebiet akustischer Wellen in vorgegebenen Bereichen erzeugt wird. Die akustischen Wellen gehen von zwei Quellen aus und breiten sich In den zu erneuernden Geweben aus, wobei eine nachfolgende natürliche Regeneration der jeweiligen biologischen Gewebe in den genannten Bereichen ermöglicht wird. Darüber hinaus werden verschiedene Ausgestaltungen der Einrichtung für eine Ausführung des Verfahrens vorgeschlagen. Um den Erneuerungseffekt verschiedener biologischer Gewebe, die unterschiedlich tief liegen, zu erreichen, werden Mikroverletzungsgebiete ohne thermische Einwirkung, d. h. ohne Verdampfung oder Koagulation aller höherliegenden Gewebe gebildet, d. h. die Regeneration der Gewebe kommt ohne jegliches Wachstum der fibrösen Zellen zustande. Folglich liegt eine tatsächliche und nicht nur visuell beobachtete Verjüngung vor.

## Beschreibung

Die Erfindung betrifft ein Verfahren zur biologischen Gewebeerneuerung nach dem Oberbegriff des Anspruchs 1 und Einrichtungen zur Ausführung des Verfahrens nach dem Oberbegriff der Ansprüche 8, 10 und 12.

Die Erfindung ist in der Medizin und auch in der Chirurgie, darunter auch in der Schönheitschirurgie, einsetzbar. Somit ist die Erfindung z. B. zur Behandlung von trophischen und lange nicht heilenden Geschwüren, Dekubitalgeschwüren, Brandverletzungen, Narben usw. geeignet. Ein weiterer Anwendungsbereich ist die Verjüngung der biologischen Gewebe, darunter der Haut, mit unterschiedlicher Lokalisation. Das Verfahren beruht auf einer Übertragung einer nichtmechanischen Energie, insbesondere akustischen Wellenenergie, in die biologischen Gewebe des menschlichen Körpers. Die Erfindung betrifft auch verschiedene Ausgestaltungen einer Einrichtung, die die zur Ausführung des Verfahrens benötigte Wellenenergie generiert.

Die Behandlungs- und Verjüngungsverfahren, welche Laserenergie, Ultraschallenergie und ähnliche Arten der nichtmechanischen Energie einsetzen, werden zurzeit in der Chirurgie, darunter auch in der Schönheitschirurgie, immer mehr verbreitet.

So Ist z. B. ein Hautverjüngungsverfahren mittels Ablation oder Vaporisation der Oberhaut mit Hilfe einer CO₂-Kohlensäuregaslaserstrahlung mit 10,6 µm langen Wellen oder einer Er-Laserstrahlung (Er: YAG) mit 2,94 µm langen Wellen (Palomar 2940 Fractional Laser, Doka SmartXide DOT, Candela C02RE) [1] bekannt. Der Heileffekt beruht In diesem Fall auf einer Verdampfung In der Oberhaut mit geringfügigem Wärmeschaden für die tiefen Hautschichten. Das führt zu keiner Volldestruktion aller Hautschichten und ruft ein neues Zellwachstum hervor. Die Mängel dieses bekannten Verfahrens sind seine hohe Traumatisierung, eine lange Rehabilitationsdauer und ein Vorhandensein einer Wundoberfläche. Dies verursacht ein hohes Ansteckungsrisiko, eine Dolenz sowohl während der Behandlung als auch im Laufe der Regenerationszeit, ein Risiko einer Pigmentierungsänderung und einer Bildung von narbigen Hautveränderungen. Darüber hinaus ist das Verfahren zur Anwendung an beweglichen Körperteilen, wie Hals, Augenlidern usw., nicht geeignet, weil der Heilungsverlauf der Wundoberfläche eine Unbeweglichkeit des Behandlungsfelds voraussetzt.

Aus dem Stand der Technik ist auch ein Verfahren einer nichtinvasiven Fotoverjüngung bekannt. Dabei dringt Strahlung tief in die Haut hinein und ruft eine Verletzung von Kollagenfasern mit nachfolgender Stimulation einer Neukollagenbildung hervor (Palomar 1540 Fractional Laser, Candela GentleMAX, Candela Smoothbeam) [2].

Dieses Verfahren bietet die Möglichkeit, praktisch beliebige Hautbereiche zu bearbeiten. Die Mängel des Verfahrens sind ein niedriger Heileffekt und eine nicht zufriedenstellende Optik. Die synthetisierte Kollagenmenge reicht nicht aus, um einen gewünschten Verjüngungseffekt zu erreichen. Unter Verjüngungseffekt wird dabei eine beobachtete Verkleinerung der Faltengröße verstanden. Nach diesem Verfahren wird die Hautfarbe dank der verbesserten Kapillardurchblutung verbessert und ein temporäres kutanes Ödem hervorgerufen, was einen provisorischen Effekt der Faltenverkleinerung schafft.

Aus dem Stand der Technik sind Hautverjüngungsverfahren unter Einsatz von nichtinvasiver Ultraschallbestrahlung bekannt [3, 4, 5]. Die genannten Verfahren ermöglichen eine Behandlung von verschiedenen Hautbereichen in vorgegebenen Einwirkungszonen. Diese Verfahren sind dadurch bemängelt, dass die Gewebe durch eine Ultraschallwellenfront beansprucht werden. Folglich wird nicht nur die vorgegebene Zone behandelt, sondern auch die Umgebungsgewebe werden betroffen. Das führt zu einer Vergrößerung der Gewebeverletzungszone und verzögert die Rehabilitation.

Der nächstkommende Stand der Technik gegenüber dem erfindungsgemäßen Verfahren ist ein Verfahren einer mikroablativen Fotoverjüngung der Haut [6]. Das Wesen dieses Verfahrens besteht darin, dass die Haut mit Laser bestrahlt wird. Bei dieser Einwirkung handelt es sich nicht um einen breiten Laserstrahl sondern um zahlreiche Mikrobündel. Dabei bewirkt jeder der Mikrobündel entweder eine Koagulation und Verdampfung oder eine Ablation der Hautmikrobereiche je nach den spektralen und zeitlichen Kenngrößen der benutzten Strahlung. Die Mikrobündel können 1 bis einige Hunderte Mikrometer im Durchmesser haben und zueinander bis einige Hunderte Mikrometer abstehen. Dem Heileffekt nach diesem Verfahren liegt die Annahme zugrunde, dass die entfernten oder zerstörten Gewebe durch neue Zellen ersetzt werden. Nach einigen Behandlungen sollte es möglich sein, die gesamte Haut durch die neue Haut im Behandlungsbereich zu ersetzen. Wird ein Mikrokoagulationseffekt der Haut unter Einsatz von Gläsern mit ER-Laser benutzt (Lasergerät "fraxel", Wellenlänge 1,54 µm), so kommt die Hitzezersetzung der Hautzellen ohne Verdampfung zustande. Wird eine solche Strahlung benutzt, die sowohl eine Verdampfung als auch eine Koagulation der Hautzellen bewirken kann (z. B. CO₂-Gaslaserstrahlung, Wellenlänge 10,64 µm), so entstehen Mikrokanäle des verdampften Gewebes mit einer sie umgebenden Koagulationszone. Wird eine ER-Laserstrahlung (mit 2,94 µm langen Wellen) benutzt, so kommt die Gewebeverdampfung in Form von Mikrokanälen ohne Koagulation der umgebenden Gewebe zustande. Dieses bekannte Verfahren hat folgende Mängel:
- Die Tiefe der Mikroverletzungen, in der ein Verjüngungseffekt erreicht werden kann, ist durch die Tiefe der Koagulation oder Ablation begrenzt. Folglich ist es nicht möglich, die Verjüngung tief in der Haut (Derma) und der Unterhaut (Hypodermis) auszuführen.
- Aufgrund der geringen Tiefe der Mikroverletzungen ist es nicht möglich, eine intensive Geweberegeneration während der Behandlung der trophischen und eitrigen Wunden usw. zu bewirken; das heißt, dass das Verfahren nicht geeignet ist, wenn eine Gewebeerneuerung ganz tief durchgeführt werden muss.
- Bei diesem Verfahren handelt es sich um ein invasives Verfahren, was ein Ansteckungsrisiko der einer Einwirkung ausgesetzten Oberfläche erhöht.
- Infolge der Gewebeentfernung kommt es zu einem Kontakt zwischen den lebendigen Geweben und der Umgebung. Das kann ein Wachstum von fibrösen Geweben anstelle einer vollständigen Erneuerung der isomorphen Gewebe verursachen.
- Die anhand der mikroablativen Methoden durchgeführten Behandlungen sind schmerzhaft und setzen somit eine Anwendung eines Anästhetikums voraus.

Es ist Aufgabe der Erfindung, ein nichtinvasives Verfahren einer biologischen Gewebe-erneuerung zu entwickeln, wobei die Leistungsmerkmale, die Eigenschaften und die Strukturen der Gewebe wiederhergestellt werden. Dafür werden Mikroverletzungsbereiche in vorgegebenen Zonen der biologischen Gewebe erzeugt, wobei eine nachfolgende natürliche Regeneration der jeweiligen biologischen Gewebe in den genannten Zonen ermöglicht wird. Dabei müssen Mikroverletzungen (Mikrozerstörungen) im biologischen Gewebe erzeugt werden, ohne dass Mikrokanäle gebildet werden, die mit einer aggressiven Umgebung kontaktieren. Das wird dadurch ermöglicht, dass ein Wachstum fibrösen Gewebes völlig vermieden wird. Eine solche Einwirkung soll es ermöglichen, die Zonen der Mikroverletzungen mit einer nachfolgenden Regeneration sowohl von Oberflächen- als auch von tiefliegendem biologischem Gewebe beliebiger Art und mit beliebiger Lokalisation zu bilden. Darüber hinaus soll das Verfahren eine Minderung von Schmerzempfindungen und eines Ansteckungsrisikos Im Vergleich zu anderen aus dem Stand der Technik bekannten, darunter auch mikroablativen, Verfahren sicherstellen.

Die gestellte Aufgabe wird durch die Merkmale des Anspruchs 1 gelöst.

Die gestellte Aufgabe wird im erfindungsgemäßen Verfahren zur biologischen Gewebeerneuerung, Wiederherstellung der Leistungsmerkmale, der Eigenschaften und der Strukturen dieser Gewebe gelöst. Dabei werden Mikroverletzungsbereiche mit der Möglichkeit einer nachfolgenden natürlichen Regeneration der jeweiligen biologischen Gewebe in vorgegebenen Bereichen der biologischen Gewebe gebildet. Dafür werden die Gewebe einer mechanischen Verletzung mit vorgegebenem Schweregrad ausgesetzt, indem wenigstens ein Interferenzgebiet akustischer Wellen In vorgegebenen Bereichen erzeugt wird. Die akustischen Wellen gehen von zwei Quellen aus und breiten sich in den zu erneuernden Geweben aus.

Im allgemeinen Fall werden beim erfindungsgemäßen Verfahren starke akustische Gleichtaktwellen auf der Oberfläche eines verjüngungsbedürftigen oder eines höherliegenden Gewebes erzeugt. Diese akustischen Gleichtaktwellen haben Solleigenschaften (Auslegungseigenschaften), insbesondere eine Sollleistung. Insbesondere eine Leistungsveränderung der akustischen Wellen ermöglicht es, dementsprechend die vorgegebene Tiefe der Mikroverletzungsbereiche zu verändern. Durch die Nutzung von Interferenz der zusammenwirkenden akustischen Wellen können die Abmessungen bei diesem Verfahren vermindert und die Lokalisation in allen Richtungen des Mikroverletzungsgebiets der Gewebe ermittelt werden. Das erlaubt es, die Effizienz der Richtwirkung wesentlich zu steigern und dabei die Rehabilitationsdauer zu verkürzen. Die Mikroverletzung der Gewebe nach dem erfindungsgemäßen Verfahren ist von nicht thermischem Charakter. Aus diesem Grund kommt bei der Einwirkung auf die biologischen Gewebe weder ihre Verdampfung noch Koagulation zustande. Außerdem sind die akustischen Wellen fähig, in die biologischen Gewebe auf eine bestimmte Tiefe einzudringen, ohne dass jegliche Kanäle gebildet werden (diese Tiefe wird durch die Eigenschaften der akustischen Wellen festgelegt). Somit wird der Kontakt zwischen den lebendigen Geweben und Sauerstoff infolge dieser Einwirkung nach dem erfindungsgemäßen Verfahren nicht verlängert. Das verringert das Risiko eines fibrösen Gewebewachstums und lässt die Annahme zu, dass es sich in diesem Fall um eine tatsächliche und keine visuell beobachtete biologische Erneuerung/Verjüngung der biologischen Gewebe handelt. Die Energie der akustischen Wellen nimmt in den Interferenzzonen mit vorgegebener Lokalisation zu. Unter der Einwirkung der akustischen Wellenenergie werden in diesen Zonen Bereiche eines verletzten und nicht zerstörten Gewebes gebildet. Die Regeneration der biologischen Gewebe kann nicht nur Infolge einer vollständigen Zerstörung der Gewebezellen sondern auch durch ihre teilweise Verletzung zustande komme. Dadurch erfolgt die biologische Gewebeerneuerung in den Gebieten mit einer vorgegebenen Lokalisation. Da es zu keiner vollständigen Zerstörung der tief liegenden Zellen der biologischen Gewebe kommt, kann auch die Rehabilitationsdauer wesentlich verkürzt werden. Die Dolenz bei der Einwirkung nach diesem erfindungsgemäßen Verfahren Ist auch wesentlich vermindert worden, weil der Verletzungsbereich kleiner Ist, der Verletzungsgrad vorgegeben werden kann und weil es keine Wärmeeinwirkung gibt.

Die Fläche der Quellen der phasengleichen akustischen Wellen liegt vorzugsweise im Bereich zwischen 10 nm² und 10 µm². Alle Nullpunkte (Zentren/Epizentren) der akustischen Wellen werden vorteilhaft im gleichen Abstand zueinander angeordnet. Diese Abstände werden in einem Bereich zwischen 10 µm und 1 cm gewählt.

Gemäß dem erfindungsgemäßen Verfahren werden die mechanischen Verletzungsbereiche der Gewebe vorteilhaft unterhalb der Oberfläche des Gewebes ausgebildet, welches im Kontakt mit der Umgebung steht, ohne dass die Kontaktfläche zwischen den lebendigen Geweben und den aggressiven Medien vergrößert wird, Somit sorgt das erfindungsgemäße Verfahren dafür, dass Mikroverletzungsbereiche erzeugt werden können, ohne dass die Berührungsfläche zwischen den lebendigen Geweben und der Umgebung vergrößert werden muss. Dadurch kann das Ansteckungsrisiko während der Rehabilitation im Vergleich zu den bekannten Verfahren wesentlich vermindert werden.

Die Mindestleistung der erzeugten akustischen Wellen wird so gewählt, dass:
- die Leistung einer von einem Zentrum/Epizentrum (Nullpunkt) ausgehenden Einzelwelle nicht ausreichend ist, um die einer Einwirkung auszusetzenden biologischen Gewebe mechanisch zu verletzen/zu zerstören;
- die bei einer Interferenz der von Nebenzentren ausgehenden Wellen erreichte Gesamtleistung dafür ausreicht, um die einer Einwirkung auszusetzenden biologischen Gewebe mit einem vorgegebenen Schweregrad mechanisch zu verletzen.

Das nach der Einwirkung auf die Hautoberfläche entstehende Erythem kann zur visuellen Kontrolle einer ausreichenden Einwirkung benutzt werden.

Bei manchen vorteilhaften Ausgestaltungen einer Einrichtung zur Ausführung des erfindungsgemäßen Verfahrens wird die Ausgangsleistung Jeder akustischen Einzelwelle so gewählt, dass die Gebiete der mechanischen Verletzung des biologischen Gewebes sowohl im Interferenzgebiet der genannten Welle mit wenigstes einer der Nebenwellen erzeugt werden, als auch in der wenigstens unmittelbar rings um den Nullpunkt der genannten akustischen Welle liegenden Zone erzeugt werden.

Die Frequenz ist eine weitere Eigenschaft der akustischen Wellen, deren Veränderung das Ergebnis der Einwirkung auf das biologische Gewebe variieren kann. So entsteht bei der Auswahl der Frequenz der akustischen Wellen je nach Eigenschwingungefrequenz der jeweiligen biologischen Gewebe die Möglichkeit, auf konkrete biologische Gewebe selektiv regenerierend einzuwirken, indem eine Resonanz mit den erwähnten Frequenzen erreicht wird. Somit wird eine solche Frequenz der akustischen Welle aus der Reihe der vorteilhaften Ausgestaltungen der Einrichtungen gemäß der Eigenfrequenz des verjüngungsbedürftigen biologischen Gewebes gewählt, welche eine selektive Einwirkung auf das genannte verjüngungsbedürftige biologische Gewebe sicherstellt.

Bei vorteilhaften Ausgestaltungen der Einrichtung wird der Grad der mechanischen Verletzung aus einem Bereich gewählt, welcher zwischen zwei Niveaus liegt, und zwar dem einer Zerstörung der Zellmembranintegrität und dem einer Volldestruktion der Zellen des verjüngungsbedürftigen biologischen Gewebes. Dadurch erlaubt die Einwirkung, eine Regeneration der biologischen Gewebe sowohl mit einer Destruktion der ganzen (intakten) Zellen dieser Gewebe als auch ohne Destruktion zu stimulieren. Dabei kann ein Erneuerungs-/Verjüngungseffekt sogar bei einer teilweisen Verletzung der Zellen der biologischen Gewebe erreicht werden.

Bei anderen vorteilhaften Ausgestaltungen werden die akustischen Wellen in Form von akustischen Richtwellen erzeugt. Dadurch wird es auch möglich, die Bildung der Mikroverietrungsbereiche zu lokalisieren und zu optimieren.

Die gestellte Aufgabe wird auch anhand der Ausgestaltungen einer Einrichtung zur Ausführung des oben beschriebenen erfindungsgemäßen Verfahrens zu einer biologischen Gewebeerneuerung gelöst. Dieses Verfahren umfasst die Wiederherstellung der Leistungsmerkmale, der Eigenschaften und der Strukturen von Geweben. Die Einrichtung enthält eine Strahlungsquelle sowie ein Mittel zur Bildung von zahlreichen Zentren/Epizentren von akustischen Wellen auf der Oberfläche der erneuerungsbedürftigen oder der höherliegenden biologischen Gewebe.

In einer ersten Ausgestaltung der Einrichtung wird die gestellte Aufgabe dadurch gelöst, dass die Strahlungsquelle in Form einer Ultraschallquelle ausgebildet ist. Die Ultraschallquelle hat ein Mittel zur Bildung von zahlreichen Zentren/Epizentren von gleich zueinander beabstandeten akustischen Wellen auf der Oberfläche der erneuerungsbedürftigen oder der höherliegenden biologischen Gewebe.

Bei einer zweiten Ausgestaltung der Einrichtung wird die gestellte Aufgabe dadurch gelöst, dass die Strahlungsquelle in Form einer Laserstrahlungsquelle ausgebildet ist, und bei dem Mittel zur Bildung von zahlreichen Zentren/Epizentren von akustischen Wellen auf der Oberfläche der erneuerungsbedürftigen oder der höherllegenden biologischen Gewebe handelt es sich um einem Stoff, welcher über einen Effekt verfügt, Wellen mit einer vorgegebenen Länge selektiv zu absorbieren. Dieser Stoff wird auf die Oberfläche des biologischen Gewebes an vorgegebenen Punktstellen mit vorgegebenen Maßen aufgetragen. Dabei liegen diese Punktstellen im gleichen Abstand zueinander.

Bei der ersten und der zweiten Ausgestaltung der Einrichtung ist es vorteilhaft, dass der Abstand zwischen den Zentren/Epizentren der akustischen Wellen im Bereich zwischen 10 µm und 1 cm liegt.

Bei einer dritten Ausgestaltung der Einrichtung wird die gestellte Aufgabe dadurch gelöst, dass die Strahlungsquelle als eine Laserstrahlungsquelle ausgebildet ist, und bei dem Mittel zur Bildung von zahlreichen Zentren/Epizentren akustischer Wellen auf der Oberfläche der erneuerungsbedürftigen oder der höherliegenden biologischen Gewebe handelt es sich um ein Mittel zur Umwandlung einer räumlichen Strahlintensitätsverteilung, Dabei wird eine periodische Struktur (Rasterstruktur) mit Maximalwerten und Minimalwerten der Lichtenergie auf der Oberfläche des genannten biologischen Gewebes gebildet. Dabei ist die Laserstrahlungsquelle so ausgebildet, dass sie eine Strahlung mit bestimmten Parametern erzeugen kann. Innerhalb dieser Parameter kann diese Strahlung durch das biologische Gewebe wirksam absorbiert werden, und die akustischen Wellen können bei den Maximalwerten der Lichtenergie erzeugt werden.

Die oben genannten sowie alle anderen Vorteile des erfindungsgemäßen Verfahrens zur biologischen Gewebeerneuerung und Wiederherstellung der Leistungsmerkmale, Eigenschaften und Strukturen der Gewebe, sowie die Vorteile der Ausgestaltungen der entsprechenden Einrichtung werden eingehend an einem Beispiel von möglichen vorteilhaften Ausgestaltungen und anhand der Figuren mit jeweiligen Bezugszeichen weiter unten betrachtet.

Diese Ausgestaltungen sind allerdings nicht auf die hier beschriebenen Fälle beschränkt. Es zeigen:
- Fig. 1: ein Übersichtsbild zur Bildung von Mikrwerletzungsbereichen der Zellen im biologischen Gewebe gemäß einer der möglichen Ausgestaltungen,
- Fig. 2: ein Übersichtsbild zur Bildung von Mikroverletzungsbereichen der Zellen im biologischen Gewebe gemäß einer zweiten Ausgestaltung,
- Fig. 3: ein Übersichtsbild zur Bildung von Mikroverletzungsbereichen der Zellen im biologischen Gewebe gemäß einer dritten Ausgestaltung und
- Fig. 4: eine schematische Darstellung der Einrichtung nach der dritten Ausgestaltung.

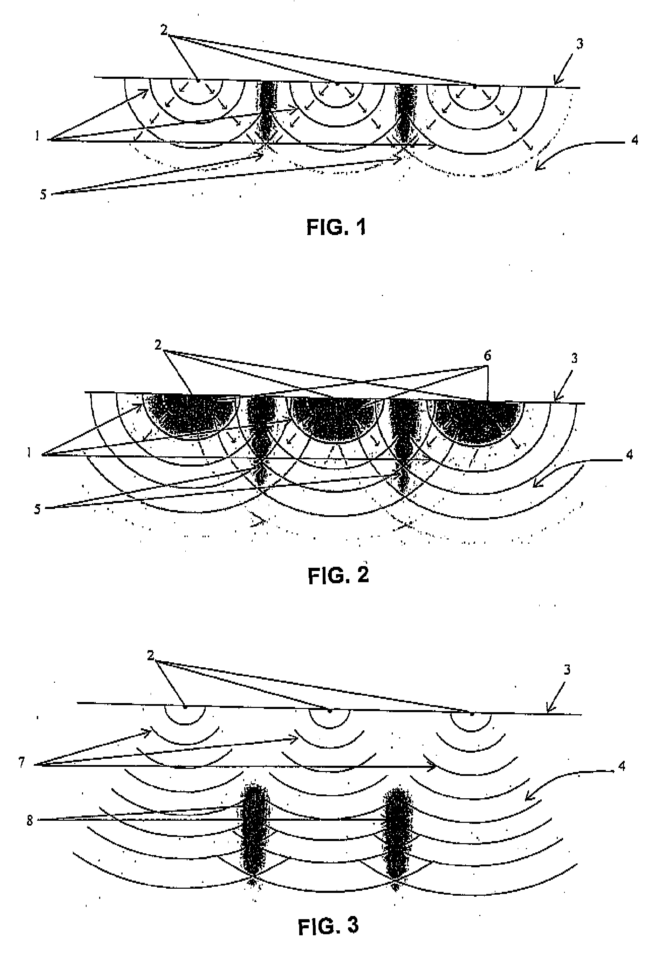

Fig. 1 weist ein Übersichtsbild zur Bildung von Mikroverletzungsbereichen der Zellen im biologischen Gewebe gemäß einer der möglichen Ausgestaltungen auf. Hier breiten sich akustische Wellen 1 von entsprechenden Zentren/Epizentren (Nullpunkten) 2 auf einer Oberfläche 3 eines biologischen Gewebes 4 ins Innere des biologischen Gewebes 4 aus. Dabei ist die Leistung Jeder der akustischen Wellen 1 so gewählt, dass diese Leistung nicht ausreicht, um jegliche Bestandteile des zu behandelnden biologischen Gewebes 4 mechanisch zu zerstören. Die Mikroverletzungsbereiche werden nur in den Interferenzzonen 5 (in der Figur dunkel gekennzeichnet) der akustischen Wellen 1 entstehen, welche den benachbarten Zentren/Epizentren 2 entspringen.

Fig. 2 zeigt ein Übersichtsbild zur Bildung von Mikroverletzungsbereichen der Zellen im biologischen Gewebe gemäß einer zweiten der möglichen Ausgestaltungen. Hier breiten sich die akustischen Wellen 1 von den entsprechenden Zentren/Epizentren 2 auf der Oberfläche 3 des biologischen Gewebes 4 ins Innere des biologischen Gewebes 4 aus. Dabei ist die Leistung jeder der akustischen Wellen 1 so gewählt, dass sie mechanische Zerstörungsbereiche 6 des zu behandelnden biologischen Gewebes 4 neben dem jeweiligen Zentrum/Epizentrum 2 (Volldestruktion der Oberflächengewebe) und Mikroverletzungsbereiche (Bereiche mit lokalen Verletzungen) in den Interferenzzonen 5 der den benachbarten Zentren/Epizentren 2 entspringenden akustischen Wellen 1 bilden wird. Die mechanischen Zerstörungsbereiche 6 und die Interferenzzonen 5 sind in der Figur dunkel gekennzeichnet.

Fig. 3 zeigt ein Übersichtsbild zur Bildung von Mikroverletzungsbereichen der Zellen im biologischen Gewebe nach einer dritten der möglichen Ausgestaltungen. Hier breiten sich akustische Wellen 1 von den jeweiligen Zentren/Epizentren 2 auf der Oberfläche 3 des biologischen Gewebes 4 ins Innere des biologischen Gewebes 4 aus. Um die Mikroverletzungsbereiche in den tief liegenden Geweben zu bilden, werden die akustischen Wellen in Form von akustischen Richtwellen 7 erzeugt. Die Mikroverletzungsgebiete bilden sich in den Interferenzzonen 8 aller zwei akustischen Richtwellen 7 von den entsprechenden benachbarten Zentren/Epizentren 2. Die Interferenzzonen 8 sind in der Figur dunkel gekennzeichnet.

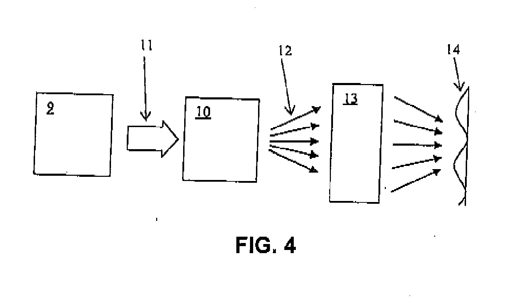

Fig. 4 zeigt eine schematische Darstellung der Einrichtung nach der dritten Ausgestaltung. Hier ist die Strahlungsquelle in Form einer Laserstrahlungsquelle 9 ausgebildet. Das Mittel zur Bildung von zahlreichen Zentren/Epizentren der akustischen Wellen auf der Oberfläche der erneuerungsbedürftigen oder der höherliegenden biologischen Gewebe enthält einen Lichtteiler 10 und ein optisches System 13. Der Lichtteiler 10 teilt das Ausgangslaserbündel 11 In so viele Laserbündel 12 auf, die erforderlich sind, um die vorgegebene Verteilung zu erreichen. Das optische System 13 bringt die mit dem Lichtteiler 10 erzeugten Laserbündel 12 unter solchen Winkeln zusammen, die erforderlich sind, um eine vorgegebene räumliche Strahlintensitätsverteilung 14 mittels einer Mehrstrahlinterferenz zu bekommen.

Das angemeldete Verfahren wird wie folgt ausgeführt:
Die akustischen Wellen 1 (7) mit vorgegebenen Eigenschaften (Leistung, Frequenz) werden mit Hilfe einer der Ausgestaltungen der erfindungsgemäßen Einrichtung zur biologischen Gewebeerneuerung mit Wiederherstellung der Leistungsmerkmale, der Eigenschaften und der Strukturen der Gewebe erzeugt. Jede akustische Welle 1 (7) fängt an, sich vom jeweiligen Zentrum/Epizentrum 2 aus ins Innere des biologischen Gewebes 4 auszubreiten. Die Mikroverletzungsbereiche werden je nach konkreter Ausgestaltung des angemeldeten Verfahrens gebildet.

So reicht z. B. die Mindestleistung der akustischen Welle 1 bei der Ausgestaltung nach Fig. 1 nicht aus, um jegliche Bestandteile des behandelbaren biologischen Gewebes 4 mechanisch zu zerstören, Deswegen ist die Ausbreitung der Welle 1 von keiner Destruktion des biologischen Gewebes 4 begleitet. Jedoch ermöglicht die Interferenz mit den aus den benachbarten Zentren/Epizentren 2 entspringenden akustischen Wellen 1, die Gesamtleistung der akustischen Welle 1 lokal so weit zu steigern, bis diese Leistungswerte ausreichen, um die Gebiete mit mechanischen Verletzungen eines bestimmten Verletzungsgrads der biologischen Gewebe 4 in diesen Interferenzzonen 5 zu bilden. Dabei werden sich diese Gebiete von der Oberfläche 3 ins Innere des Gewebes ausbreiten und Zonen mit ungleichmäßiger Mikroverletzung (eines vorgegebenen Verletzungsgrads) der Zellen im biologischen Gewebe 4 darstellen.

Eine solche Verletzung der Gewebe setzt keine Vergrößerung der Kontaktfläche zwischen den lebendigen Geweben und der aggressiven Umgebung voraus, was das Wachstum des fibrösen Gewebes minimiert.

Nimmt die Leistung der akustischen Wellen 1 zu, so können solche Leistungswerte nach Ausgestaltung gemäß Fig. 2 erreicht werden, bei denen jede akustische Einzelwelle 1 fähig ist, eine unabhängige Destruktion der Gewebe hervorzurufen. Dabei wird sich der Einwirkungsprozess etwas ändern und wie folgt aussehen. Die Wellen 1 gehen von jedem Zentrum/Epizentrum 2 aus und dringen ins Innere des biologischen Gewebes 4 ein. Sie verursachen eine mechanische Zerstörung (Zonen 6 der mechanischen Zerstörung), bis die Leistungswerte dieser Wellen unter ihre Schwellenwerte sinken. Die weitere Ausbreitung der Wellen 1 ins innere des biologischen Gewebes 4 wird von keiner Gewebezersetzung begleitet bis auf die Interferenzzonen 5 der Wellen 1, welche von den benachbarten Zentren/Epizentren 2 ausgehen. Somit handelt es sich beim Verletzungsgebiet um einen Volldestruktionsbereich des Oberflächengewebes (Zonen 6 der mechanischen Zerstörung) und um Bereiche mit lokalen Verletzungen (Interferenzzonen 5).

Der visuell beobachtete Effekt wird sich in diesem Fall dadurch bemerkbar machen, dass der sogenannte "Frost" auf der zu behandelnden Oberfläche entsteht. Beim "Frost"-Effekt handelt es sich um Gebiete mechanisch zerstörten Gewebes. Dabei ermöglicht die Leistungsvariation der akustischen Welle, auch die Tiefenlage der Mikroverletzungsbereiche zu variieren.

Um die Mikroverletzung der tiefliegenden biologischen Gewebe 4 und die nachfolgende Regeneration der Gewebe zu erreichen, wird die Einwirkung gemäß der dritten Ausgestaltung (s. Fig. 3) des erfindungsgemäßen Verfahrens eingesetzt. Das heißt, es werden akustische Richtwellen 7 erzeugt. In diesem Fall wird die Interferenz der akustischen Wellen 7 nur in der Tiefe der biologischen Gewebe zustande kommen, ohne dass die Oberflächenschichten verletzt werden. Die Gebiete der mechanischen Mikroverletzung werden in den Interferenzzonen 8 entstehen.

Die Regeneration der Gewebe nach dem erfindungsgemäßen Verfahren wird im Vergleich zum Prototyp schneller verlaufen, denn sogar bei der Einwirkung nach der zweiten Ausgestaltung (s. Fig. 2) werden nur Oberflächengewebe (Zonen 6 der mechanischen Zerstörung) eine vollständige mechanische Destruktion erfahren. Die tief im Gewebe liegenden Zellen werden nicht vollständig zerstört, sondern nur verletzt (Interferenzzonen 5). Bei allen anderen Ausgestaltungen einschließlich beliebiger hier nicht näher betrachteter Ausgestaltungen liegt überhaupt keine Volldestruktion jeglicher biologischer Gewebe vor.

Die Einrichtung für die Ausführung des erfindungsgemäßen Verfahrens zur biologischen Gewebeerneuerung kann auch verschiedene Ausgestaltungen haben.

Die Einrichtung nach der ersten Ausgestaltung kann als Strahlungsquelle eine leistungsstarke Ultraschallquelle aufweisen. Als ein "Erzeuger" der Zentren/Epizentren 2 der akustischen Wellen 1 (7) kann eine Kontaktfläche benutzt werden. Die Kontaktfläche wird in Form eines geordneten Nadelsatzes ausgebildet. Die Nadeln werden in einem vorgegebenen Abstand zueinander angeordnet (der Nadelabstand liegt im Bereich zwischen 10 µm und 1 cm) und berühren die Oberfläche 3 des biologischen Gewebes 4 über 10 nm² bis 10 µm² große Flächen.

Bei der Strahlungsquelle gemäß der Einrichtung nach der zweiten Ausgestaltung kann es sich um einen leistungsstarken Laserimpulsgenerator handeln. Als "Erzeuger" der Zentren/Epizentren 2 der akustischen Wellen 1 (7) kann ein spezielles Absorptionsmedium (Absorptionsstoff) mit hohen Absorptionszahlen für die gewählte Strahlungswellenlänge benutzt werden. Dieses Absorptionsmedium wird auf die zu behandelnde Oberfläche aufgetragen. Dabei kann der Absorptionsstoff in Form von Einzelpunkten mit Punktflächen im Bereich zwischen 10 nm² und 10 µm² und mit Punktabständen im Bereich zwischen 10 µm und 1 cm aufgetragen werden.

Bei der Strahlungsquelle gemäß der Einrichtung nach der dritten Ausgestaltung kann es sich um einen leistungsstarken Generator für Laserstrahlung handeln, welche durch das zu behandelnde biologische Gewebe gut absorbiert wird. In diesem Fall brauchen keine zusätzlichen Stoffe auf die zu behandelnde Oberfläche 3 aufgetragen werden. Das Laserbündel muss aber auf solche Weise quer verteilt werden, dass diese Querverteilung eine Rasterstruktur mit Energiemaximalwerten und - minimalwerten auf der der Einwirkung ausgesetzten Oberfläche bildet. Die Flächen der Hochenergiegebiete müssen dabei im Bereich zwischen 10 nm² und 10 µm² liegen (und die Abstände dazwischen müssen im Bereich zwischen 10 µm und 1 cm sein). Somit liegt der Einrichtung nach der dritten Ausgestaltung eine Mehrstrahlinterferenz zugrunde. Bei dieser Einrichtung ist die Umwandlung eines breiten Ausgangslaserstrahls in die vorgegebene endgültige räumliche Intensitätsverteilung auf der Oberfläche des einwirkungsbedürftigen oder des höherliegenden biologischen Gewebes vorgesehen.

Der Übersichtsplan dieser Einrichtung ist Fig. 4 zu entnehmen. Das durch die Laserquelle 9 erzeugte breite Laserbündel 11 fällt auf einen Lichtteiler 10 ein. Der Lichtteiler 10 teilt das Laserbündel 11 in mehrere Laserbündel 12 auf. Dabei handelt es sich um eine solche Menge von Laserbündeln 12, welche erforderlich ist, um eine vorgegebene Verteilung zu erreichen. Das optische System 13 bringt die anhand des Lichtteilers 10 erzeugten Laserbündel 12 unter solchen Winkeln zusammen, die erforderlich sind, um eine vorgegebene räumliche Bündelintensitätsverteilung 14 mittels der Mehrstrahlinterferenz zu erreichen.

Beim Lichtteiler 10 handelt es sich um ein optisches Element oder eine Einrichtung, welches/welche das Ausgangslaserbündel 11 in die vorgegebene Menge der Laserbündel 12 mit bestimmter Sollintensität aufteilt. Der Lichtteiler 10 kann unter Einsatz folgender Baugruppen oder ihrer Kombinationen ausgebildet werden:
- ein Phasengitter oder Amplitudengitter (Beugungsgitter) oder kompliziertere Beugungselemente,
- ein Spiegel mit dielektrischer Schicht oder Metallüberzug, Metallspiegel,
- Kugellinsen und/oder Zylinderlinsen mit dielektrischer Schicht oder Metallüberzug oder ohne Beschichtung,
- Prismen mit dielektrischer Schicht oder Metallüberzug oder ohne Beschichtung,
- Mikrolinsen- und Mikroprismenanlagen mit dielektrischer Schicht oder ohne Beschichtung.

Beim optischen System 13 handelt es sich um ein optisches Element oder um eine Einrichtung, welche die anhand des Lichtteilers 10 in der Ebene der endgültigen räumlichen Bündelintensitätsverteilung 14 erzeugten Laserbündel 12 zur Deckung bringt. Das optische System stellt sicher, dass das Laserbündel auf die Ebene der endgültigen räumlichen Bündelintensitätsverteilung 14 unter solchen Winkeln einfällt, die erforderlich sind, um die vorgegebene endgültige räumliche Bündelintensitätsverteilung 14 zu erreichen. Das optische System 13 kann unter Einsatz folgender Baugruppen oder ihrer Kombinationen ausgebildet werden:
- Kugellinsen und/oder Zylinderlinsen mit dielektrischer Schicht oder Metallüberzug oder ohne Beschichtung, Spiegel mit dielektrischer Schicht oder Metallüberzug, Metallspiegel,
- Prismen mit dielektrischer Schicht oder Metallüberzug oder ohne Beschichtung.

Bei der endgültigen räumlichen Bündellntensitätsverteilung 14 handelt es sich um ein geordnetes Rastersystem (periodisches System). Das Rastersystem besteht aus interferenzmaximalwerten und -minimalwerten der Intensität eines Bündels mit vorgegebenen Maßen und Periodizität in der Ebene der Oberfläche 3 des behandelbaren oder des höherliegenden biologischen Gewebes 4.

Die Einrichtung nach der dritten Ausgestaltung gemäß Flg. 4 arbeitet In einem breiten Spektralbereich der Wellenlängen von 200 nm bis 20 µm und in einem breiten Bereich der kennzeichnenden Maximum- oder Minimumwerte der räumlichen Bündelintensitätsverteilung 14 und zwar im Bereich zwischen der Wellenlänge der benutzten Strahlung und 1 mm.

Der Er: YAG-Laser arbeitet mit einer Wellenlänge von 2,94 µm. Um insbesondere die Er:YAG-Laserstrahlung umzuwandeln, kann ein Lichtteiler 10 verwendet werden. Der Lichtteiler 10 besteht aus zwei Phasen-Beugungsgittern. Die Phasen-Beugungsgitter werden aus geschmolzenem Quarz KI mit einem "U-förmigen" Strichprofil gefertigt. Die Gitter werden so ausgerichtet, dass ihre Striche normal zueinander gerichtet sind. Die Strichtiefe wird so gewählt, dass das Diffraktionsbild keine nullte Beugungsordnung aufweist. Dann entfallen 80 % der Ausgangsstrahlungsenergie (Energie des Bündels 11) auf die vier Bündel 12 der ersten Beugungsordnung. Diese Laserbündel 12 liegen paarweise in normal zueinander liegenden Ebenen (in gegenseitig um 90 Grad versetzen Ebenen). Die Beugungsordnungen über den vier Laserbündeln 12 der ersten Beugungsordnung werden mit einer speziellen Blende ausgefiltert.

Das aus zwei Linsen bestehende optische System 13 bringt die resultierenden Laserbündel 12 in der Ebene der endgültigen räumlichen Bündelintensitätsverteilung 14 zusammen.

Im Endeffekt wird die räumliche Bündelintensitätsverteilung 14 in Form von den sich abwechselnden Maximalwerten und Minimalwerten erreicht. Die resultierende intensitätsvertellung 14 entlang der Linien, welche mit der Ausrichtung der Gitter des Lichtteilers 10 zusammenfallen, ist eine sinusartige Intensitätsvertellung mit einer 100 µm langen Periode.

Die Verfahren und die entsprechenden Einrichtungen zur Erneuerung/Verjüngung der biologischen Gewebe sind In der medizinischen Praxis bekannt. Dennoch zeigt die oben angeführte Beschreibung samt den Beispielen, welche einige nicht erschöpfende Ausgestaltungen der Erfindung schildern, dass es das erfindungsgemäße Verfahren und die Einrichtung hierfür ermöglichen, neue unerwartete technische Ergebnisse zu erreichen. Das hängt vor allem damit zusammen, dass der Erneuerungseffekt verschiedener biologischer Gewebe, die unterschiedlich tief liegen, dadurch geschaffen werden kann, dass die Mikroverletzungsgebiete ohne thermische Einwirkung, d. h. ohne Verdampfung oder Koagulation aller höherliegenden Gewebe gebildet werden können. Somit kommt die Regeneration der Gewebe ohne jegliches Wachstum fibröser Zellen zustande. Folglich liegt eine tatsächliche und nicht nur visuell beobachtete Verjüngung vor.

### Informationsquellen

1. B. Yeremeyev, K. Kalaydzyan. Laser gegen Falten. Elektronischer Almanach, "Kosmetik und Medizin". [Elektronische Informationsressource] - 6. April 2012 - http://daniel.ru/cm/arc/r403.htm.
2. Palomar-Verjürtgung. Web-Site des GesundheitsZentrum/Epizentrums RODEN [Elektronische Informationsressource] - 4. Mai 2012 - http://www.roden.by/cosmetology/palomar-omolojente/.
3. Patentanmeldung US 2011/0218464 A1, veröffentlicht am 08.09.2011.
4. Patentanmeldung US 2012/0016239 A1, veröffentlicht am 19.01.2012.
5. Patentanmeldung US 2012/0053458 A1, veröffentlicht am 01.03.2012.
6. Patent US 6,997,923 B2, veröffentlicht am 31.10.2002.

## Patentansprüche

1. Verfahren zur biologischen Gewebeerneuerung mit Wiederherstellung der Leistungsmerkmale, der Eigenschaften und der Strukturen dieser Gewebe, indem In vorgegebenen Bereichen der biologischen Gewebe Mikroverletzungsbereiche mit der Möglichkeit einer nachfolgenden natürlichen Regeneration der jeweiligen biologischen Gewebe in den genannten Bereichen gebildet werden,
**dadurch gekennzeichnet,**
**dass** die Gewebe einer mechanischen Verletzung mit einem vorgegebenen Schweregrad ausgesetzt werden, indem wenigstens ein Interferenzgebiet (5) akustischer Wellen (1) in vorgegebenen Bereichen erzeugt wird und
**dass** die akustischen Wellen (1) dabei von zwei Quellen ausgehen und sich in den zu erneuernden Geweben (4) ausbreiten.

2. Verfahren nach Anspruch 1,
**dadurch gekennzeichnet,**
**dass** alle Zentren (2) der akustischen Wellen (1) im gleichen Abstand zueinander angeordnet werden, wobei diese Abstände aus einem Bereich zwischen 10 µm und 1 cm gewählt werden,

3. Verfahren nach einem der Ansprüche 1 oder 2,
**dadurch gekennzeichnet,**
**dass** die mechanischen Verletzungsbereiche der Gewebe (4) unterhalb der Oberfläche (3) des Gewebes (4) ausgebildet werden, welches im Kontakt mit der Umgebung steht, ohne dass die Kontaktfläche zwischen den lebendigen Geweben und den aggressiven Medien vergrößert wird.

4. Verfahren nach einem der Ansprüche 1 bis 3,
**dadurch gekennzeichnet,**
**dass** die Ausgangsleistung jeder akustischen Einzeiwelle (1) so gewählt wird, dass die Gebiete der mechanischen Verletzung des biologischen Gewebes (4) sowohl in einem Interferenzgebiet (5) der genannten Welle (1) mit wenigstes einer der Nebenwellen als auch in einer wenigstens unmittelbar rings um den Nullpunkt der genannten akustischen Welle (1) liegenden Zone erzeugt werden.

5. Verfahren nach einem der Ansprüche 1 bis 4,
**dadurch gekennzeichnet,**
**dass** die Frequenz der akustischen Welle, welche eine selektive Einwirkung auf das genannte verjüngungsbedürftige biologische Gewebe (4) sicherstellt, gemäß der Eigenfrequenz des verjüngungsbedürftigen biologischen Gewebes (4) gewählt wird.

6. Verfahren nach einem der Ansprüche 1 bis 5,
**dadurch gekennzeichnet,**
**dass** der Grad der mechanischen Verletzung aus einem Bereich gewählt wird, welcher zwischen zwei Niveaus liegt, und zwar dem der Zerstörung (6) der Zellmembranintegrität und dem der Volldestruktion (5) der Zellen des verjüngungsbedürftigen biologischen Gewebes.

7. Verfahren nach einem der Ansprüche 1 bis 6,
**dadurch gekennzeichnet,**
**dass** die akustischen Wellen (1) in Form von akustischen Richtwellen (7) erzeugt werden.

8. Einrichtung für eine Ausführung des Verfahrens zur biologischen Gewebeerneuerung mit Wiederherstellung der Leistungsmerkmale, der Eigenschaften und der Strukturen von Geweben nach einem der Ansprüche 1 bis 7,
**dadurch gekennzeichnet,**
**dass** sie eine Strahlungsquelle in Form einer Ultraschallquelle sowie ein Mittel zur Bildung von zahlreichen Zentren (2) akustischer Wellen (1) auf der Oberfläche (3) der erneuerungsbederftigen oder der höherliegenden biologischen Gewebe (4) aufweist, wobei diese Zentren/Epizentren (2) im gleichen Abstand zueinander liegen.

9. Einrichtung nach Anspruch 8,
**dadurch gekennzeichnet,**
**dass** der Abstand zwischen den Zentren (2) der akustischen Wellen (1) im Bereich zwischen 10 µm und 1 cm liegt.

10. Einrichtung für eine Ausführung des Verfahrens zur biologischen Gewebeerneuerung mit Wiederherstellung der Leistungsmerkmale, Eigenschaften und Strukturen der Gewebe nach einem der Ansprüche 1 bis 7,
**dadurch gekennzeichnet,**
**dass** sie eine Laserstrahlungsquelle (9) sowie ein Mittel zur Bildung von zahlreichen Zentren (2) akustischer Wellen (1) auf der Oberfläche (3) der erneuerungsbedürftigen oder der höherliegenden biologischen Gewebe (4) aufweist, wobei dieses Mittel aus einem Stoff besteht, welcher über einen Effekt verfügt, Wellen mit einer vorgegebenen Wellenlänge selektiv zu absorbieren und
**dass** dieser Stoff auf die Oberfläche des biologischen Gewebes (4) an vorgegebenen Punktstellen mit vorgegebenen Maßen aufgetragen wird, wobei diese Punktstellen im gleichen Abstand zueinander liegen.

11. Einrichtung nach Anspruch 10,
**dadurch gekennzeichnet,**
**dass** der Abstand zwischen den Zentren (2) der akustischen Wellen (1) im Bereich zwischen 10 µm und 1 cm liegt.

12. Einrichtung für eine Ausführung des Verfahrens zur biologischen Gewebeerneuerung mit Wiederherstellung der Leistungsmerkmale, Eigenschaften und Strukturen der Gewebe nach einem der Ansprüche 1 bis 7,
**dadurch gekennzeichnet,**
**dass** sie eine Laserstrahlungsquelle (9) sowie ein Mittel zur Bildung von zahlreichen Zentren (2) akustischer Wellen (1), auf der Oberfläche (3) der erneuerungsbedürftigen oder der höherliegenden biologischen Gewebe (4) aufweist, dass dieses Mittel (10, 11, 12, 14) als ein Mittel zur Umwandlung räumlicher Strahlintensitätsverteilung ausgebildet Ist, wobei eine periodische Struktur mit Maximalwerten und Minimalwerten der Lichtenergie auf der Oberfläche (3) des genannten biologischen Gewebes (4) gebildet wird,
**dass** die Laserstrahlungsquelle (9) so ausgebildet wird, dass sie die Strahlung mit bestimmten Parametern erzeugt,
**dass** diese Strahlung Innerhalb dieser Parameter dabei durch das biologische Gewebe (4) wirksam absorbiert wird und
**dass** die akustischen Wellen (1) bei den Maximalwerten der Lichtenergie erzeugt werden.
